# EUROPEAN PATENT SPECIFICATION

(11) **EP 0 416 419 B1**
(45) Date of publication and mention of the grant of the patent: **08.11.1995**
(21) Application number: 90116383.2
(22) Date of filing: 27.08.1990
(51) Int. Cl.: C12M 1/40, C12Q 1/00, C12M 1/34

(54) **Immobilized alcohol oxidase, method for its preparation and measuring apparatus**
Immobilisierte Alkoholoxydase, Methode zu deren Herstellung und Messgerät
Alcool-oxydase immobilisée, méthode pour sa préparation et appareil de mesure

(30) Priority: 25.08.1989 JP 219104/89; 12.03.1990 JP 61703/90
(43) Date of publication of application: 13.03.1991
(73) Proprietor: NEW OJI PAPER CO., LTD., Tokyo (JP)
(72) Inventor: Hashizume, Yoshio, Kakogawa-shi, Hyogo-ken 675 (JP); Kariyone, Akio, Nishinanajo, Shimogyo-ku, Kyoto 600 (JP); Hayashi, Ryuzo, Higashiosaka-shi, Osaka 577 (JP); Oka, Minako, /Gorindanchi H-2-63, Minami-ku, Sapporo-shi, Hokkaido 005 (JP)
(74) Representative: Beetz & Partner Patentanwälte

(56) References cited:
- EP-A- 0 325 404
- EP-A- 0 326 081

## Description

This invention relates to a stable immobilized alcohol oxidase, a method for preparing it, and its use, preferably in a highly accurate and stable alcohol measuring apparatus. The invention further relates to a highly accurate and stable alcohol measuring apparatus which can measure glucose at the same time.

Measuring apparatus using an immobilized enzyme are characterized by convenience, expedition, and substrate specificity, and are applied in various fields such as clinical analysis, food analysis, and environmental instrumentation.

Improved devices of this kind of measuring apparatus using an immobilized enzyme, and particularly an alcohol dehydrogenase for ethanol analysis, are required in many technical fields, such as food and fermentation technology, and in the clinical field. However, with respect to the enzyme used for the measurement of alcohol, no sufficient stability has yet been obtained.

Known enzymes which can be used for alcohol measurement are alcohol dehydrogenase (EC 1.1.1.1) and alcohol oxidase (EC 1.1.3.13).

However, the alcohol dehydrogenase requires NAD (nicotinamide adenine dinucleotide) as a coenzyme, leading to higher cost of reagents used in analysis, and the stability of the enzyme is low. Further, the use of this enzyme is limited to measurements in solution.

On the other hand, alcohol oxidase is known to have been used for measurements after immobilization.

However, such conventional immobilized alcohol oxidase is relatively low in activity, and the stability is unsatisfactory for practical use.

US-A-4 556 635, for example, describes a method for measuring the alcohol concentration in water immiscible organic systems. The preferable temperature is about 25 °C because this enzyme is easily deactivated at relatively high temperatures. Thus, using a conventional immobilization method, measuring methods using alcohol oxidase are substantially limited in their practical use.

US-A-4 900 666 discloses a measuring method and a test device using alcohol oxidase. However, as described in this specification, the alcohol oxidase is denaturated very readily; therefore, various stabilizers were being studied, however, stabilizers having sufficient effect for application to a measuring apparatus using immobilized alcohol oxidase have not yet been obtained.

In addition, these measuring apparatus using conventional immobilized enzyme suffer from the defect that after a high alcohol concentration exceeding the measuring range has been measured, the response to low alcohol concentrations becomes smaller, that is, the sensitivity is not constant.

In the field of fermentation and foods, there is a demand for the simultaneous measurement of glucose, in the form of sweeteners or of raw material, and of alcohol, as a product or as main component. However, use of the conventional unstable immobilized alcohol oxidase could not afford development of a satisfactory simultaneous measuring apparatus.

Accordingly, it is the object of the present invention to provide a stable immobilized alcohol oxidase and a high-accuracy and stable alcohol measuring apparatus using it and particularly, to provide an alcohol measuring apparatus with excellent stability at room temperature or higher and of no sensitivity variation after measurement of high alcohol concentrations. Furthermore, a high-accuracy and stable alcohol measuring apparatus which can measure glucose at the same time, shall be provided.

The above object is achieved according to the independent claims. The dependent claims relate to preferred embodiments.

The immobilized alcohol oxidase of the present invention is obtainable by at least the steps of
(A) Treating the surface of a carrier, preferably a porous carrier, with
   an aminosilane
   coupling agent, to form amino groups on the carrier surface;
(B) bonding a multifunctional aldehyde to amino groups on the carrier surface formed in step (A),
(C) washing the carrier to remove unreacted multifunctional aldehyde,
   and
(D) bonding an alcohol oxidase to aldehyde groups of the multifunctional aldehyde bond to the amino groups,
wherein
- a diatomaceous earth or a fire brick or fire brick material is used as carrier,
   and
- γ-aminopropyltriethoxysilane, 4-aminobutyldimethylmethoxysilane and/or 4-aminobutyltriethoxysilane are used as aminosilane coupling agents.

The method of the present invention for preparing immobilized alcohol oxidase comprises at least the steps of
(A) Treating the surface of a carrier, preferably a porous carrier, with
   an aminosilane
   coupling agent, to form amino groups on the carrier surface;
(B) bonding a multifunctional aldehyde to amino groups on the carrier surface formed in step (A),
(C) washing the carrier to remove unreacted multifunctional aldehyde,
   and
(D) bonding an alcohol oxidase to aldehyde groups of the multifunctional aldehyde bond to the amino groups;
it is characterized in that
- a diatomaceous earth or a fire brick or fire brick material is used as carrier,
   and
- γ-aminopropyltriethoxysilane, 4-aminobutyldimethylmethoxysilane and/or 4-aminobutyltriethoxysilane are used as aminosilane coupling agents.

EP-A-325 404 discloses a method of immobilizing physiologically active substances such as enzymes on an inorganic support, wherein the inorganic support is treated with a specific aminosilane coupling agent for modifying the support with primary amino group containing molecules, and then the amino groups are caused to react with a dialdehyde such as glutaraldehyde, whereupon the enzyme is immobilized thereon through free aldehyde groups. According to this document, a specific aminoalkylaminopropyltrialkoxysilane of the formula

(RO)₃SiCH₂CH₂CH₂NH(CH₂)ₙNH₂

is used wherein R is C₁₋₄-alkyl, and n is an integer of 5 to 12.

It is stated in this prior art document (column 2, lines 27 to 35) that this specific mediator molecule leads to less interference with the immobilized physiologically active substance as compared with 3-aminopropyltriethoxysilane or N-(2-aminoethyl)-3-aminopropyltrimethoxysilane used heretofore.

The present independent claims have been delimited vis-à-vis this prior art.

Preferably, said carrier is a fire brick or fire brick material obtained by high temperature treatment of diatomaceous earth containing material.

In a preferred embodiment of the invention, the alcohol oxidase is immobilized on a carrier having at least a porous surface, in the form of a thin layer having a thickness corresponding to approximately the thickness of one molecule of alcohol oxidase (monolayer).

The invention also provides a flow type alcohol measuring apparatus comprising a column filled with an immobilized alcohol oxidase provided at the upstream side, and a hydrogen peroxide electrode provided downstream of said column, which is characterized by an immobilized alcohol oxidase according to the present invention.

In a preferred embodiment of the invention, the flow type measuring apparatus additionally comprises an immobilized enzyme electrode for detecting glucose concentration provided upstream of the column or downstream of the hydrogen peroxide electrode.

According to the invention, an immobilized enzyme having higher heat resistance and longer life time is obtained.

According to the invention, the alcohol oxidase is stably immobilized so that an excellent alcohol measuring apparatus may be easily composed. Particularly, alcohol measuring apparatus with higher heat resistance and with practically no variation of sensitivity even after the measurement of high alcohol concentrations can be composed.

According to the invention, alcohol and glucose measuring apparatus of high-accuracy and stability can be realized.

In the following, the invention will be described with more details with reference to the drawings:
- Figures 1a - 1d: are schematic representations for explaining the preparation of the immobilized alcohol oxidase of the present invention according to the embodiment of Example 1;
- Figure 2: is a block diagram of a flow-type measuring apparatus used in Examples 1 - 3;
- Figure 3: is a diagram showing the measurement results of Example 1 and Comparative Examples 1 and 2;
- Figure 4: is a diagram showing the measurement results of Example 2;
- Figure 5: is a diagram showing the measurement results of Example 3;
- Figure 6: is a block diagram showing the flow-type glucose and alcohol measuring apparatus according to an embodiment of the invention used in Example 4,
and
- Figure 7: is a diagram showing the calibration curves of glucose and of ethanol in Example 4.

While studying the characteristics of the alcohol oxidase and testing various immobilizing methods, it has been found experimentally that in measurements using an immobilized alcohol oxidase electrode, after measurement of a sample solution of high alcohol concentration, the response to a low-concentration alcohol solution sample becomes smaller.

### Experimental Results

Alcohol oxidase was mixed with bovine serum albumin and further with glutaraldehyde and placed on a platinum electrode to give an immobilized enzyme electrode. Measurement was made using a three electrode system using this enzyme electrode as a working electrode, a counter-electrode of platinum wire and an Ag/AgCl reference electrode. In a sodium phosphate buffer solution of pH 7.5, a voltage of +0.6 V against the Ag/AgCl reference electrode was applied to the working electrode. The measuring temperature was room temperature (25 °C). First the sensitivity was recorded with ethanol equivalent to 0.1 - 1 mM added to the buffer solution. In this concentration range, the output current was proportional to the concentration of ethanol added. Then, ethanol equivalent to 1 M was added and left to stand for one minute, and the buffer solution was replaced by a new one, and the sensitivity was checked again with ethanol of low concentration. This showed that the sensitivity was decreased to about 50 %.

However, the enzyme electrode thus exposed to ethanol of such a high concentration restores its sensitivity to about 90 % of the initial value when allowed to stand about 1 h in buffer solution.

Then, the sensitivity was measured one minute after addition of hydrogen peroxide equivalent to 10 mM. This showed a sensitivity decrease and then sensitivity restoring with time, similar to the case of exposure to the high concentration alcohol solution.

The above experimental results show that the alcohol oxidase is inhibited by H₂O₂ produced, and aldehyde which is also produced by the catalytic reaction of the alcohol oxidase can cause deactivation of the alcohol oxidase.

To prevent deactivation by such products, hydrogen peroxide or the like should be removed from the vicinity of the immobilized alcohol oxidase as quickly as possible.

The present invention is based on the results of various investigations in this respect.

In the invention, an alcohol oxidase is preferably immobilized on a carrier in the form of a thin layer having a thickness corresponding to approximately a thickness of one molecule of the alcohol oxidase, namely a single layer of about 20 to 300 Å in thickness, corresponding to the diameter of the alcohol oxidase having a molecular mass of about 200 000.

Here, "a single layer" of the alcohol oxidase means that the alcohol oxidase molecules are not arranged in multilayers on the carrier surface.

Thus, the thickness of the layer of an immobilized alcohol oxidase is preferably about 20 to 300 Å, corresponding to the diameter of the alcohol oxidase.

Since the alcohol oxidase is thus arranged in a single layer, reaction products are readily removed from the enzyme layer, and a deactivation of the alcohol oxidase by reaction products can be prevented.

The alcohol oxidase which may be used in the present invention is e.g. an enzyme produced by microorganisms such as Basidiomycetes and yeast, and is contained particularly in large quantities in the peroxysomes of methanol-utilizing yeasts.

Carriers having a porous surface are preferably used. Further among these, carriers having relatively large pores and a specific surface area of 2 to 9 m²/g are preferably used. The carrier used is more preferably a fire brick or fire brick material obtained by high temperature treatment of a diatomaceous earth containing material.

The alcohol oxidase is immobilized through the functional groups of the activated carrier. To introduce these functional groups, multifunctional aldehydes are used.

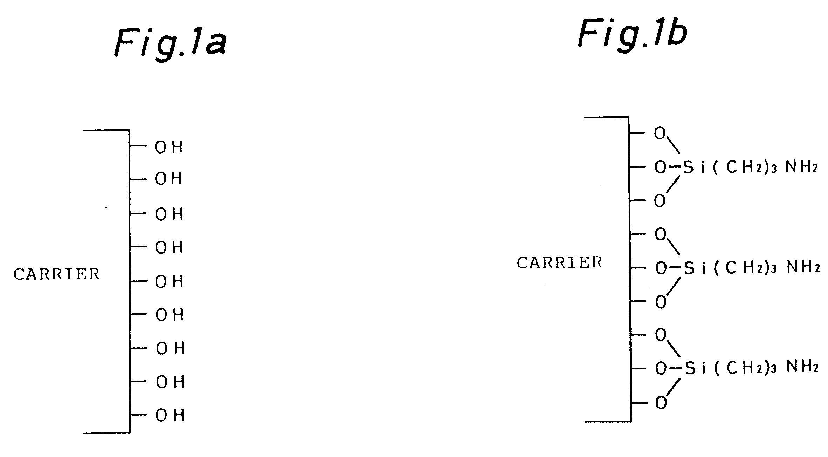

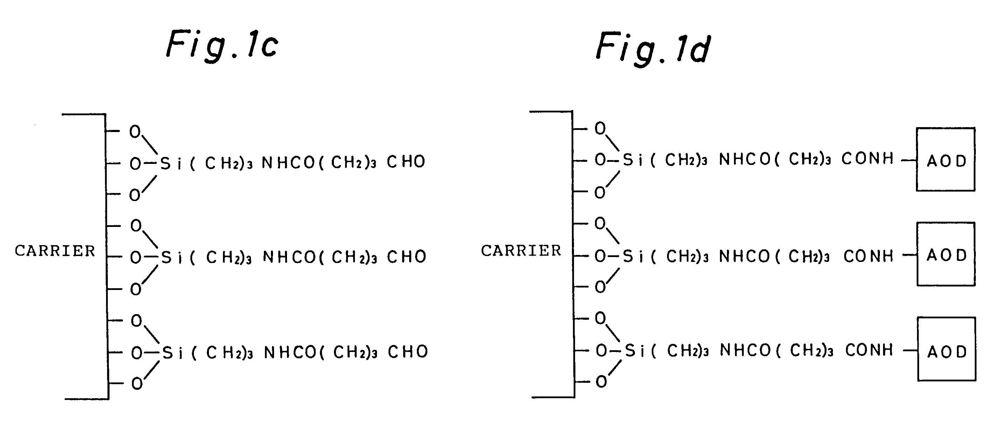

According to the present method, the following advantages are achieved:
(1) There is no possibility of formation of multiple layers in step (A) of providing an amino group comprising layer (silane coupling agent layer) on the carrier surface (see Fig. 1b);
(2) There is no possibility of formation of multiple layers also in step (B) of reacting the multifunctional aldehyde such as glutaraldehyde (see Fig. 1c);
(3) Since the cross-linking reaction is performed with the alcohol oxidase brought into contact after free, excessive cross-linking/coupling agent has been removed, the alcohol oxidase molecules are not cross-linked such that they are arranged into multilayers. That is, when the alcohol oxidase is immobilized by use of the cross-linking agent, a plurality of cross-linking positions on the carrier surface may react with the alcohol oxidase, but alcohol oxidase molecules are not piled on other alcohol oxidase molecules (see Fig. 1d).

For these reasons, a thin layer of alcohol oxidase can be formed on the carrier surface and can effectively prevent the remaining of hydrogen peroxide, etc. in the interior of the enzyme layer, thus preventing the deactivation of the alcohol oxidase by reaction products.

The amount of enzyme immobilized on the carrier is estimated by the enzyme specific activity per unit surface area of the carrier. In the case of the present invention, the activity of alcohol oxidase is preferably 10 to 1000 IU/m² (International Units/m²). If a thin layer of immobilized enzyme is formed, an excellent immobilized enzyme both in sensitivity and life expectancy is obtained with such amounts of immobilized enzyme.

According to the preferred embodiment, the use of the diatomaceous earth based silicate carrier makes it possible to immobilize enzyme on the carrier having fairly coarse pores on the surface. Therefore, both the alcohol, the substrate, and hydrogen peroxide, the product, can be removed without remaining in the pores. Thus, inhibition of the alcohol oxidase is prevented.

The diatomaceous earth based silicate carrier is exemplified by natural diatomaceous earth and its granulates, and fire brick and fire brick materials, obtained by high temperature treatment of diatomaceous earth. Suitable fire brick materials may also be obtained by high temperature treatment of the material mixed with about 0.1 to 10 % of flux, such as, sodium carbonate, magnesium carbonate, calcium carbonate, etc., in addition to the diatomaceous earth. Any of these are aggregates of primary particles of about 1 µm, having relatively coarse pores with a specific surface area of 2 to 9 m²/g. This value shows that the pores (0.1 µm or more) verified by scanning electron microscopy are main pores.

The investigation of the lifetime of the alcohol oxidase immobilized on various carriers, including diatomaceous earth based silicate carriers and silica gel, having various pore diameters, showed that those immobilized on a diatomaceous earth based silicate carrier were the most excellent in heat resistance and had long lifetimes.

The reason therefor is probably the quick product removal effect with larger pore diameters, and there is also the possibility that trace amounts of calcium and iron contained in the diatomaceous earth based silicate carriers may have a stabilizing effect, or the silane coupling agent is relatively firmly bonded to decrease the dropout of the enzyme.

The aminosilane coupling treatment is carried out with use of γ-aminopropyltriethoxysilane, 4-amino-butyldimethylmethoxysilane and/or 4-aminobutyltriethoxysilane as silane coupling agents, generally by immersing the carrier in a silane coupling agent solution in dry benzene or anhydrous toluene.

The multifunctional aldehyde is exemplified by glutaraldehyde and glyoxal. In the practical use, bifunctional aldehydes, particularly glutaraldehyde, which is a mild cross-linking agent, are preferable.

The alcohol oxidase is deactivated even by glutaraldehyde, a relatively mild cross-linking agent. In the above method, however, since the alcohol oxidase is immobilized by contact after the silane coupling agent and the multifunctional aldehyde have been bonded to the carrier, the enzyme is not deactivated by unnecessary modification by excessive free aldehyde.

Thus, the concept of the present invention provides an immobilized alcohol oxidase having excellent heat resistance and long lifetime.

The immobilized alcohol oxidase according to the invention can be used also as a reactor for aldehyde production and for removal of alcohol, however, it is preferably used for alcohol measuring apparatus.

As an alcohol measuring apparatus, a column reactor system is preferable for preventing inhibition by reaction products and for attaining a long life.

As measuring apparatus using immobilized enzyme, amperometric measuring apparatus are available. Such a system measures the increase or decrease of the electrode active substance caused by the enzyme reaction by means of the variation of the current output from the immobilized enzyme electrode supplied with a constant voltage. Because of easily raised sensitivity and excellent stability, this arrangement is used in various electrodes, devices, and methods. Representative examples for amperometric analysis include those by means of an oxygen electrode and by means of a hydrogen peroxide electrode, the latter method using hydrogen peroxide electrode being better in response speed and S/N ratio.

Accordingly, the invention provides a flow-type alcohol measuring apparatus which is characterized in that a column filled with the immobilized alcohol oxidase is provided upstream side, and a hydrogen peroxide electrode is arranged downstream of the column.

With a combination of this measuring system comprising the immobilized alcohol oxidase filled column and the hydrogen peroxide electrode, and a glucose measuring electrode, a simultaneous alcohol and glucose measuring apparatus for very quick and high accuracy measurement can be composed.

Accordingly, the invention also provides a flow-type alcohol and glucose measuring apparatus which is characterized in that a column filled with the immobilized alcohol oxidase is provided upstream side, a hydrogen peroxide electrode is arranged downstream of the column, and an electrode for measuring glucose is provided in series connection.

The electrode for measuring glucose is, for example, a hydrogen peroxide electrode or an oxygen electrode having an immobilized glucose oxidase or pyranose oxidase membrane, and the hydrogen peroxide electrode is as mentioned before.

The electrode for measuring glucose can be arranged either upstream of the immobilized alcohol oxidase column or downstream of the hydrogen peroxide electrode, but in the measurement of samples containing high concentrations of alcohol and glucose, a remarkable consumption of dissolved oxygen and formation of hydrogen peroxide occur in the immobilized alcohol oxidase column. Therefore, the glucose measuring electrode is preferably arranged upstream of the immobilized alcohol oxidase column. It is of course possible to make up a double flow-path type flow measuring apparatus, but it is preferable for measurements of higher accuracy to configure a series arrangement type apparatus, since in a double flow-path type apparatus, the flow dividing ratio for the division of the injected sample flow can be largely vary due to stained piping, etc.

It is also possible to make up various simultaneous measuring units by using together an immobilized alcohol oxidase electrode other than a glucose measuring electrode. For example, a simultaneous measuring apparatus for saccharides such as cane sugar, fructose, and maltose, and organic acids such as lactic acid, and amino acids such as glutamic acid may be composed. Further, a pH electrode and an ion-selective electrode such as a sodium electrode and an ammonia electrode can be used.

In the following, the invention will be described with more details by way of examples. The given percentage values are on mass basis (mass-%).

### Example 1

### (1) Electrode Preparation

A platinum wire of 2 mm in diameter was coated with heat-shrinkable PTFE (Teflon ®), and its end was finished smoothly by filing and with No. 1500 emery paper. Using this platinum wire as the working electrode, a 1 cm² platinum plate as the counter electrode, and a saturated calomel electrode (hereinafter referred to as SCE) as the reference electrode, electrolysis was performed in 0.1 M sulfuric acid at +2.0 V for 10 minutes. After thorough washing, the platinum wire was dried at 40 °C for 10 minutes, immersed in a 10 % γ-aminopropyltriethoxysilane solution in anhydrous toluene, and washed.

20 mg of bovine serum albumin (Fraction V, supplied by Sigma Co.) was dissolved in 1 ml of distilled water, and then glutaraldehyde was added to make the resulting solution to contain 0.2 % of glutaraldehyde. 5 µl of this mixed solution were quickly put on the platinum wire prepared above, and dried and cured at 40 °C for 15 minutes to form a hydrogen peroxide selectively permeable membrane. This electrode was used as the hydrogen peroxide electrode.

### (2) Preparation of Immobilized Enzyme Column

Figs. 1a - 1d schematically show the immobilization of alcohol oxidase. As the carrier fire brick, a diatomaceous earth based silicate, was used (Fig. 1a). A quantity of 150 mg of fire brick (60 - 80 mesh classification) was thoroughly dried and allowed to stand for 1 h after addition of 1 ml of 10 % γ-aminopropyltriethoxysilane solution in anhydrous toluene. After thoroughly washing the excess of this silane coupling agent with toluene and methanol, this carrier was dried at 120 °C for 2 h (Fig. 1b). After being allowed to cool, it was allowed to stand for 1 h after addition of 0.5 ml of 5 % aqueous glutaraldehyde solution. This carrier was then thoroughly washed with water. Finally it was washed with sodium phosphate buffer solution of pH 7.0, and the buffer solution was removed as far as possible (Fig. 1c).

To this aminosilane and aldehyde treated carrier, 50 µl of an enzyme solution of alcohol oxidase (liquid enzyme specimen derived from Pichia pastolis, supplied by Sigma Co.) diluted by 10 times with sodium phosphate buffer solution of pH 7.0 was added and left to stand for 1 h under ice cooling. Then, it was thoroughly washed with the buffer solution (Fig. 1d).

The surface area of the used fire brick was 0.3 m², the acitivity of the immobilized alcohol oxidase was 62.5 IU, and accordingly, the specific activity of the immobilized alcohol oxidase was 208 IU/m². Thus, a 20 - 300 Å thick single layer was formed with nearly every alcohol oxidase molecule (shown by AOD in Fig. 1d) immobilized one by one. This carrier immobilized enzyme was filled in a polytetrafluoroethylene resin tube of an outer diameter of 3 mm, an inner diameter of 2 mm, and a length of 10 cm.

### (3) Measuring Apparatus

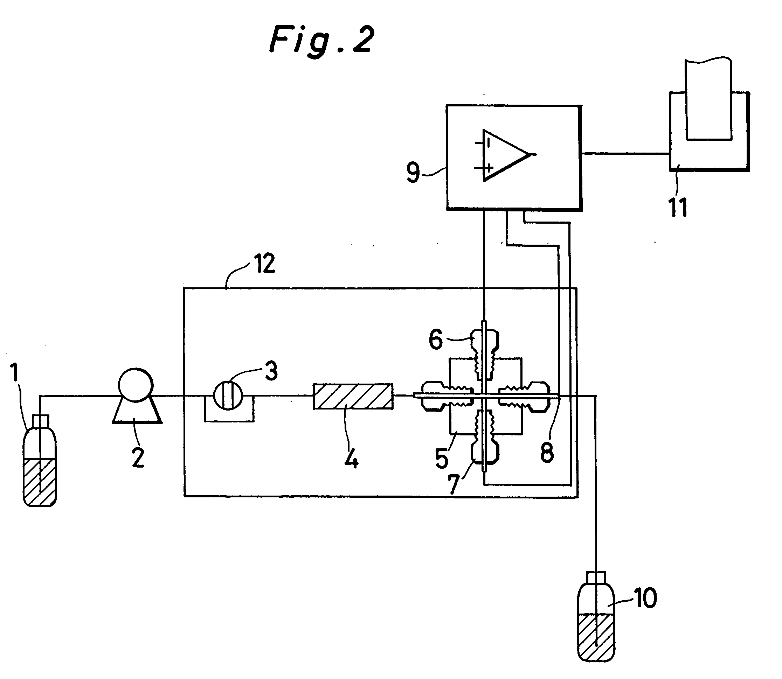

A flow-type measuring apparatus shown in Fig. 2 was used. This flow-type measuring apparatus comprised an injector 3 for high-speed liquid chromatography (7125 type injector made by Rheodyne Co.) and the measuring cell 5 with stainless steel piping containing the hydrogen peroxide electrode 6, the Ag/AgCl electrode 7 as a reference electrode and the counterelectrode 8. The injector 3, the immobilized enzyme column 4, and the measuring cell 5 were connected in this order with PTFE (Teflon ®) piping of an inner diameter of 0.5 mm and a length of 0.5 m. The internal volume of the measuring cell 5 was 40 µl, and the hydrogen peroxide electrode 6 and the Ag/AgCl electrode 7 were arranged opposite to each other and connected through the pipe of buffer solution. A voltage of +0.6 V against the Ag/AgCl electrode 7 was applied to the hydrogen peroxide electrode 6 by means of a potentiostat 9. The injector 3, the immobilized enzyme column 4, and the measuring cell 5 were installed in a thermostat 12. The temperature in the thermostat 12 was held at 37 ± 0.2 °C. For pumping the buffer solution 1, a pump 2 for high-speed liquid chromatography was used, and the buffer solution was pumped at a flow rate of 1.0 ml/min. The buffer solution was a 100 mM sodium phosphate (pH 7.0) solution containing 1 mM sodium azide.

The buffer solution used for the measurement was caught in a waste reservoir 10. The measurements were recorded by a recorder 11.

### (4) Measuring Method

The column containing the immobilized enzyme carrier was mounted immediately after immobilization on the measuring apparatus, and after the constant temperature equilibrium of the thermostat was reached, 10 µl of a 100 mM ethanol solution were injected. The sensitivity at that time was recorded. The thermostat was held at 37 °C for 8 h, and the ethanol solutions of 30 samples were measured in 1 h. During the night, the pumping was stopped, and the ambient temperature of the column was held at 30 °C.

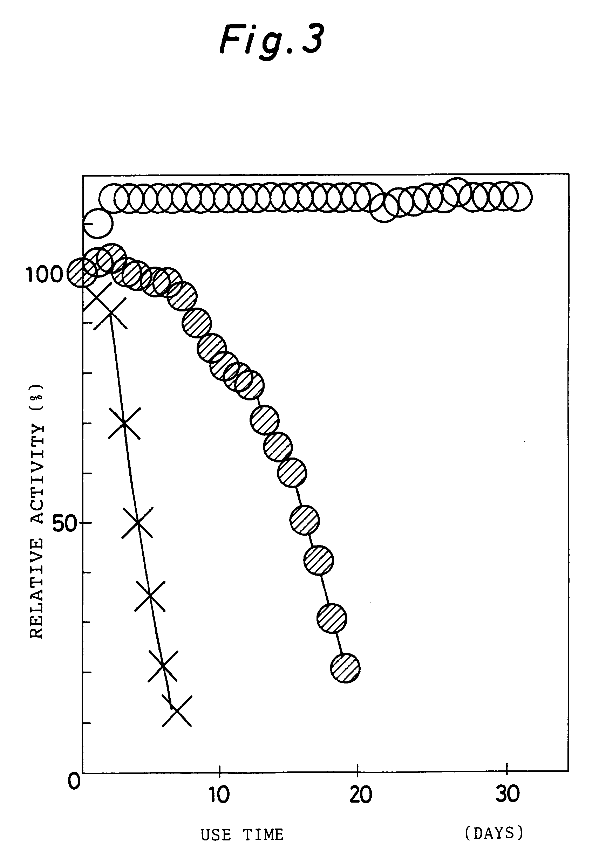

In the daytime, the measurement at 37 °C was continued for 8 h, and during the night the column was left to stand in the environment of 30 °C, and the sensitivity to the 100 mM ethanol solution was recorded every day. Making the sensitivity first recorded immediately after the immobilization 100 % (relative activity), the change of sensitivity thereafter was measured; the results are shown in Fig. 3 (o mark). For several days after the start of recording, an increase of sensitivity was observed which stabilized thereafter. This increase of the sensitivity was due to the deactivation of the catalase which was contained in the alcohol oxidase as an impurity. That is, the hydrogen peroxide which is produced simultaneously with the oxidation of alcohol is decomposed by catalase in the early stage of the measurements, but because of the deactivation of the catalse in a few days, hydrogen peroxide is quantitatively eluted from the column and detected.

As shown in Fig. 3, the immobilized alcohol oxidase prepared according to the invention was very stable with a measurement temperature of 37 °C and a storage temperature of 30 °C, showing no deterioration of the activity even after one month.

Into the apparatus provided with the immobilized enzyme, 100 mM and 1 M ethanol solutions were alternately injected. In the case of the 1 M solution, the output value was saturated and could not be determined, but had no influence on the measurement of 100 mM ethanol solution injected immediately after, and a sensitivity variation was not observed.

### Comparative Example 1

### (1) Electrode Preparation

A hydrogen peroxide electrode was prepared and used as in Example 1.

### (2) Preparation of Immobilized Enzyme Column

150 mg of fire brick (60 - 80 mesh classification) with addition of 50 µl of alcohol oxidase (liquid enzyme specimen derived from Pichia pastolis, supplied by Sigma Co.), 50 mg of bovine serum albumin, and 0.95 ml of sodium phosphate buffer solution of pH 7.0, and further with addition of glutaraldehyde to make the resulting concentration thereof 0.2 %, was left to stand for 5 h in a refrigerator to dryness. After dryness was reached, it was thoroughly washed with buffer solution. This carrier immobilized enzyme was filled into a polytetrafluoroethylene resin tube of an outer diameter of 3 mm, an inner diameter of 2 mm, and a length of 10 cm. Immobilized by this method, a relatively thick layer of the immobilized enzyme of several to several tens of microns in thickness was formed, in contrast to the thickness of 20 - 300 Å of the immobilized enzyme layer according to the invention.

### (3) Measuring Apparatus

The same apparatus as in Example 1 was used except that the above column was used as the immobilized enzyme column.

### (4) Measuring Method

The time dependence of the relative activity of the immobilized enzyme was evaluated by the same measuring method as in Example 1. The results are shown in Fig. 3 ( 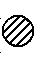 mark).

As is obvious from Fig. 3, the immobilized alcohol oxidase prepared by the method of Example 1 was very stable, while when the immobilized enzyme column obtained by the method of Comparative Example 1 was used, the sensitivity began to drop after one week, and it dropped to about 20 % of the initial sensitivity after 19 days.

When the immobilized enzyme column of Comparative Example 1 was used, the time until the hydrogen peroxide detecting peak disappeared was about 1.5 times as long as that of Example 1; this means that the retention time of hydrogen peroxide in the immobilized enzyme layer is much longer in this case.

Like in Example 1, 100 mM and 1 M ethanol solutions were alternately injected into the apparatus provided with this immobilized enzyme column. The measured values of the 100 mM ethanol solution injected after 1 M solution were decreased to about 60 %, showing a large influence of the 1 M solution measurement.

### Comparative Example 2

### (1) Electrode Preparation

A platinum wire of 2 mm in diameter was coated with heat shrinkable PTFE (Teflon ®), and its end was finished smoothly by filing and with No. 1500 emery paper. Using this platinum wire as the working electrode, a 1 cm² platinum plate as the counterelectrode, and a SCE as the reference electrode, electrolysis was performed in 0.1 M sulfuric acid at + 2.0 V for 10 minutes. After thorough washing, the platinum wire was dried at 40 °C for 10 minutes, immersed in a 10 % γ-aminopropyltriethoxysilane solution in anhydrous toluene, and washed.

20 mg of bovine serum albumin (Fraction V, supplied by Sigma Co.) were dissolved in 1 ml of distilled water, and then glutaraldehyde was added to make the resulting solution to contain 0.2 % of glutaraldehyde. 5 µl of this mixed solution were quickly put on the platinum wire prepared above, and dried and cured at 40 °C for 15 minutes. This electrode was used as the hydrogen peroxide electrode.

Further, to 50 µl of alcohol oxidase (same enzyme specimen as in Example 1) and 50 mg of bovine serum albumin, 0.95 ml of 100 mM sodium phosphorate buffer solution (pH 7.0) was added, and glutaraldehyde was further added to make the resulting mixed solution to contain 0.2 % of glutaraldehyde. 5 µl of this mixed solution were put onto the bovine serum albumin membrane of the hydrogen peroxide electrode and dried at 40 °C for 15 minutes to get an enzyme electrode for alcohol measurement. The surface area of the electrode was 3.14 x 10⁻⁶ m², the activity of the immobilized alcohol oxidase was 0.313 IU, and accordingly, the specific activity of the immobilized alcohol oxidase was 99500 IU/m². The activity was extremely high in comparison with the immobilized enzyme on the carrier in Examples 1, 2 and 3. This enzyme electrode was used in the following experiment.

### (2) Measuring Apparatus

The same apparatus as in Example 1 was used except that the enzyme electrode was used instead of the hydrogen peroxide electrode, and the immobilized enzyme column was removed.

### (3) Measuring Method

The time dependence of the relative activity was evaluated in the same manner as in Example 1. The results are shown in Fig. 3 (x mark).

In the case of the enzyme electrode, a remarkable sensitivity drop was observed from 3 days after the start of the measurements, and in 5 days the sensitivity dropped to about 35 %. It is obvious that the performance is inferior to that of the column reactor system of Example 1.

### Comparative Example 3

### (1) Electrode Preparation

The hydrogen peroxide electrode was prepared by the same method as in Example 1.

### (2) Preparation of Immobilized Enzyme Column

200 mg of silica gel (80 - 100 mesh classification) were thoroughly dried and after addition of 1 ml of 10 % γ-aminopropyltriethoxysilane solution in anhydrous toluene, were allowed to stand for 1 h. After thoroughly washing excessive silane coupling agent with toluene and methanol, this carrier was dried at 120 °C for 4 h. After being allowed to cool and addition of 0.5 ml of aqueous 5 % glutaraldehyde solution, it was allowed to stand for 1 h at room temperature. This carrier was thoroughly washed with water. Finally it was washed with sodium phosphate buffer solution of pH 7.0, and the buffer solution was removed as far as possible.

50 µl of alcohol oxidase solution (liquid enzyme specimen derived from Pichia pastolis, supplied by Sigma Co.) was diluted by 10 times with sodium phosphate buffer solution of pH 7.0 and added to the aminosilane coupled carrier, which then was left to stand for 1 h under ice cooling. Then, it was thoroughly washed with buffer solution. The surface area of the used silica gel was 24 m², the activity of the immobilized alcohol oxidase was 62,5 IU, and accordingly, the specific activity of the immobilized alcohol oxidase was 2.6 IU/m². This carrier immobilized alcohol oxidase was filled in a polytetrafluoroethylene resin tube of an outer diameter of 3 mm, an inner diameter of 2 mm, and a length of 10 cm.

### (3) Measuring Apparatus

The same apparatus as in Example 1 was used.

### (4) Measuring Method

The time dependence of the relative activity of the immobilized enzyme was checked by the same method as in Example 1.

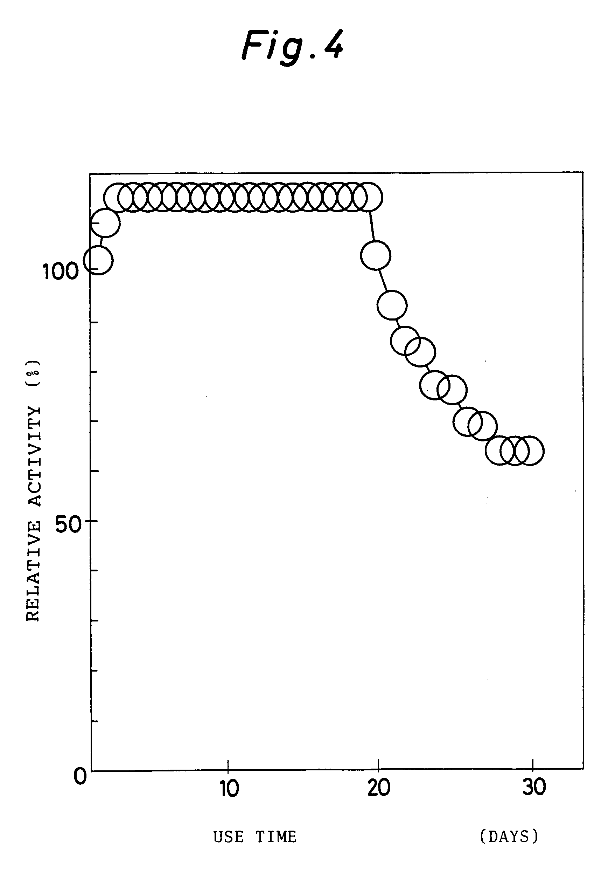

In the daytime, the measurements at 37 °C were continued for 8 h, and during the night, the column was left to stand in the environment of 30 °C. The sensitivity to the 100 mM ethanol solution was recorded everyday. The change of sensitivity is shown in Fig. 4, where the sensitivity recorded first immediately after the immobilization is 100 %. For several days after the start of recording an increase of sensitivity was observed, which then stabilized thereafter like in the case of Example 1.

As is obvious from Fig. 4, the immobilized alcohol oxidase prepared according to this Comparative Example 3 was very stable, showing no deterioration of the activity for about 3 weeks. This means that the durability is improved in comparison with Comparative Examples 1 and 2. However, the pore size of silica gel is finer than that of fire brick, which leads to a tendency of delayed elution of hydrogen peroxide, and for this reason, hydrogen peroxide may be present near the enzyme for a longer time than in the case of the fire brick carrier. This may explain the somewhat shorter lifetime of the immobilized alcohol oxidase.

Into this apparatus provided with the immobilized enzyme column, ethanol solutions of 100 mM and 1 M were alternately injected. In the case of the 1 M solution, the output value was saturated and could not be determined, but had no influence on the measurements with 100 mM ethanol solution injected immediately after.

### Example 2

### (1) Electrode Preparation

The hydrogen peroxide electrode was prepared by the same method as in Example 1.

### (2) Preparation of Immobilized Enzyme Column

150 mg of diatomaceous earth (Celite 545, 40 - 60 mesh classification, supplied by Kishida Chemical Ltd.), a diatomaceous earth based silicate carrier, were thoroughly dried and allowed to stand for 1 h with addition of 1 ml of 10 % γ-aminopropyltriethoxysilane solution in anhydrous toluene. After thoroughly washing excessive silane coupling agent with toluene and methanol, this carrier was dried at 120 °C for 4 h. After being allowed to cool, the carrier was allowed to stand for 1 h with addition of 0.5 ml of 5 % aqueous glutaraldehyde solution at room temperature. This carrier was thoroughly washed with water and finally washed with sodium phosphate buffer solution of pH 7.0, and then the buffer solution was removed as far as possible.

50 µl of enzyme solution of alcohol oxidase (liquid enzyme specimen derived from Pichia pastolis, supplied by Sigma Co.) were diluted by 10 times with sodium phosphate buffer solution of pH 7, and added to this aminosilane treated carrier and left to stand for 1 h under ice cooling. Then, it was thoroughly washed with the buffer solution. The surface area of the used diatomaceous earth was 0.3 m², the activity of the immobilized alcohol oxidase was 62.5 IU, and accordingly, the specific activity of the immobilized alcohol oxidase was 208 IU/m². This carrier immobilized enzyme was filled in a polytetrafluoroethylene resin tube of an outer diameter of 3 mm, an inner diameter of 2 mm, and a length of 10 cm.

### (3) Measuring Apparatus

The same apparatus as in Example 1 was used.

### (4) Measuring Method

The time dependence of the relative activity of the immobilized enzyme was checked by the same method as in Example 1.

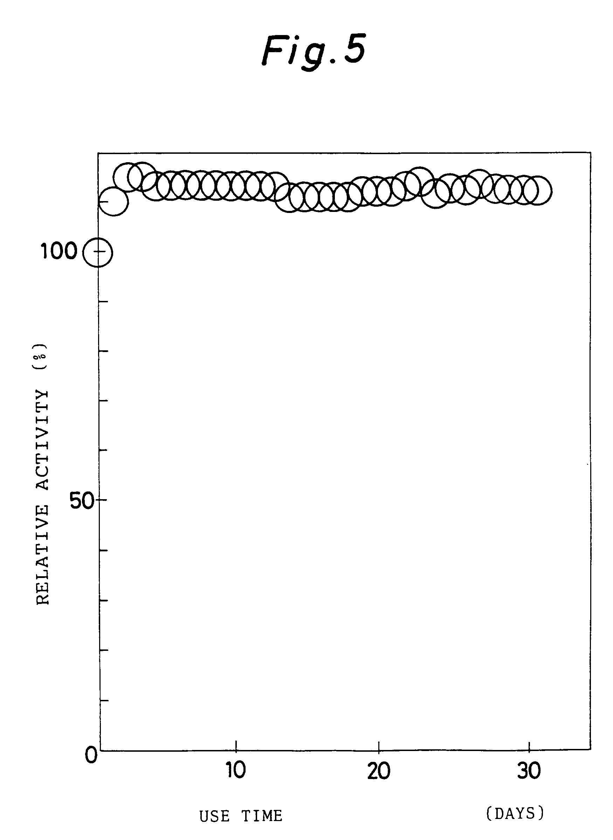

In the daytime, the measurements at 37 °C were continued for 8 h, and during the night, the column was left to stand in the environment of 30 °C. The sensitivity to 100 mM ethanol solution was recorded everyday. The change of sensitivity is shown in Fig. 5, where the sensitivity recorded first immediately after the immobilization is 100 %. For several days after the start of recording, an increase of sensitivity was observed, which then stabilized thereafter like in the case of Example 1.

As is obvious from Fig. 5, the immobilized alcohol oxidase prepared according to the invention was very stable, showing no deterioration of the activity even after one month. This shows that the stability is excellent like in Example 1.

Into this apparatus provided with the immobilized enzyme column, ethanol solutions of 100 mM and 1 M were alternately injected. In the case of the 1 M solution, the output value was saturated and could not be determined, but had no influence on the measurements with 100 mM ethanol solution injected immediately after.

### Example 3

### (1) Electrode Preparation

The hydrogen peroxide electrode and the column were prepared in the same manner as Example 1.

A glucose measuring electrode was prepared by immobilizing glucose oxidase on the selectively hydrogen peroxide permeable membrane of the same hydrogen peroxide electrode as that used in Example 1. 5 mg/ml of glucose oxidase (derived from Aspergillus niger, Type II, supplied by Sigma Co.) and 5 mg/ml of bovine serum albumin (Fraction V, supplied by Sigma Co.) were mixed in the ratio of 1:1, and glutaraldehyde was added to the mixed solution to make the resulting concentration thereof 0.2 %. After dropping 5 µl of the mixture on the selectively hydrogen peroxide permeable membrane it was dried at 40 °C for 15 minutes.

### (2) Measuring Apparatus

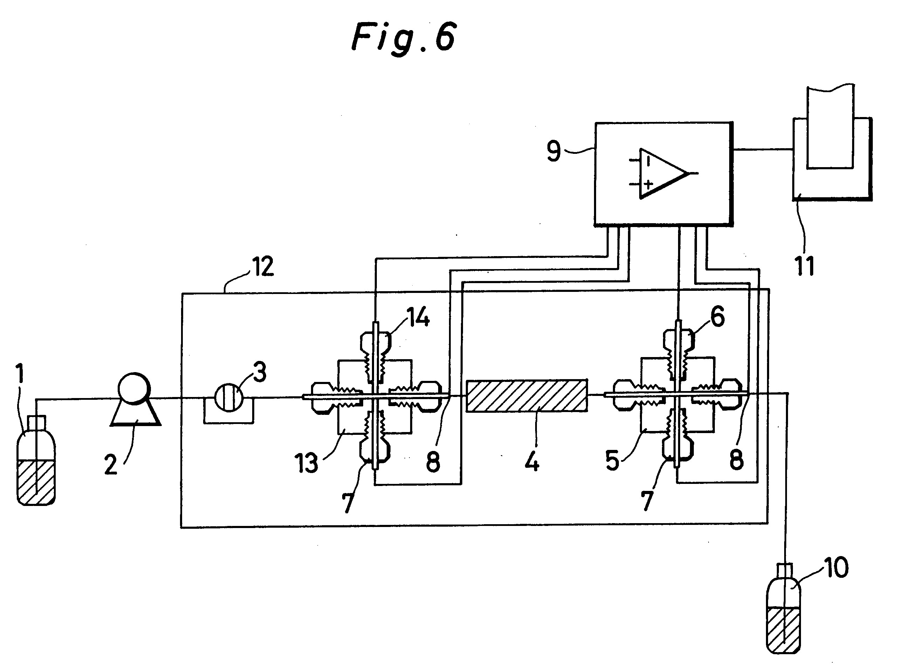

The glucose measuring electrode 14 mounted on the measuring cell 13 was attached to the flow-type measuring apparatus shown in Fig. 2. The configuration is shown in Fig. 6. The other measuring conditions were the same as in Example 1.

### (3) Measuring Method

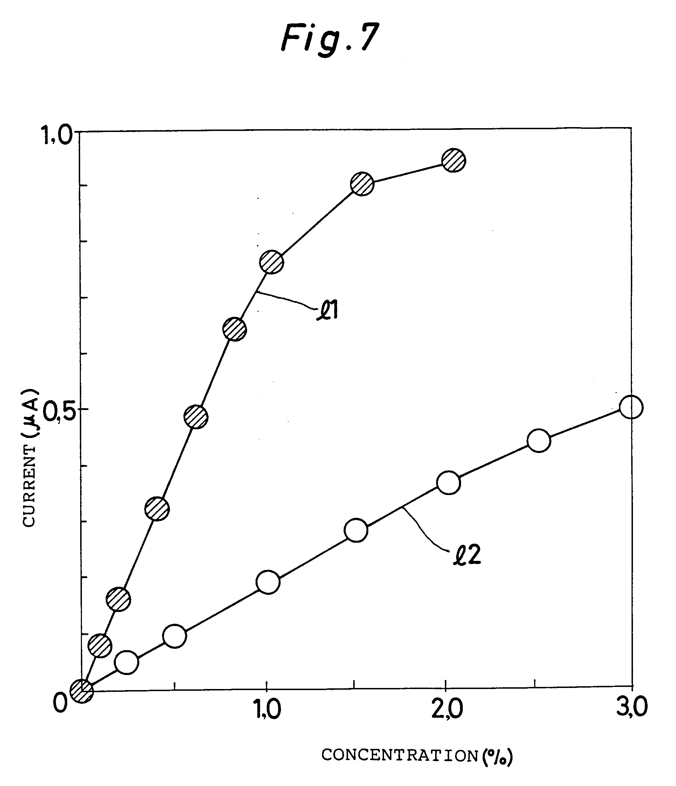

The calibration curve obtained by injecting 5 µl of ethanol solutions and of glucose solutions of various concentrations from the injector 3 is shown in Fig. 7. The calibration curve ℓ1 represents that of glucose. It is a straight line within the range up to 0.75 % (W/V). The calibration curve ℓ2 represents that of ethanol, and it is a straight line within the range up to 2.0 % (V/V). Even when a mixed solution of glucose and alcohol was injected, no interference with each other was observed.

The glucose measuring electrode showed no deterioration of sensitivity for 6 months. Thus, it was found that the apparatus according to the invention provides high-accuracy and stable glucose and alcohol measurements.

## Claims

1. A method for preparing immobilized alcohol oxidase, comprising at least the steps of
(A) Treating the surface of a carrier, preferably a porous carrier, with
an aminosilane
coupling agent, to form amino groups on the carrier surface;
(B) bonding a multifunctional aldehyde to amino groups on the carrier surface formed in step (A),
(C) washing the carrier to remove unreacted multifunctional aldehyde,
and
(D) bonding an alcohol oxidase to aldehyde groups of the multifunctional aldehyde bond to the amino groups,
characterized in that
- a diatomaceous earth or a fire brick or fire brick material is used as carrier,
and
- γ-aminopropyltriethoxysilane, 4-aminobutyldimethylmethoxysilane and/or 4-aminobutyltriethoxysilane are used as aminosilane coupling agents.

2. The method according to claim 1, characterized in that a fire brick or fire brick material obtained by high temperature treatment of diatomaceous earth containing material is used as carrier.

3. The method according to claim 1 or 2, characterized in that bifunctional aldehydes are used as multifunctional aldehydes.

4. The method according to one of claims 1 to 3, characterized in that glyoxal and/or glutaraldehyde are used as multifunctional aldehydes.

5. Immobilized alcohol oxidases, obtainable by the method according to one of claims 1 to 4, preferably in the form of an enzyme monolayer bonded to the carrier surface.

6. Use of the immobilized alcohol oxidase according to claim 5 for aldehyde production, for alcohol removal, and/or for measuring alcohol concentrations.

7. Flow-type alcohol measuring apparatus, comprising a column (4) filled with immobilized alcohol oxidase, and a hydrogen peroxide electrode (6) provided downstream of the column (4), characterized by an immobilized alcohol oxidase according to claim 5.

8. The flow-type alcohol measuring apparatus according to claim 7, characterized in that it additionally comprises an immobilized enzyme electrode (14) for detecting glucose concentration provided upstream of the column (4) with immobilized alcohol oxidase or downstream of the hydrogen peroxide electrode (6).

## Patentansprüche

1. Verfahren zur Herstellung von immobilisierter Alkoholoxidase,
das mindestens folgende Schritte umfaßt:
(A) Behandlung der Oberfläche eines Trägers, vorzugsweise eines porösen Trägers, mit einem Aminosilan-Kupplungsmittel zur Erzeugung von Aminogruppen auf der Oberfläche des Trägers,
(B) Bindung eines multifuktionellen Aldehyds an in Schritt (A) erzeugten Aminogruppen auf der Oberfläche des Trägers,
(C) Waschen des Trägers zur Entfernung von nicht umgesetztem multifunktionellem Aldehyd
und
(D) Bindung einer Alkoholoxidase an Aldehydgruppen des an den Aminogruppen gebundenen multifunktionellen Aldehyds,
gekennzeichnet durch Verwendung
- von Diatomeenerde, Schamottestein oder einem Schamottesteinmaterial als Träger und
- von γ-Aminopropyltriethoxysilan, 4-Aminobutyldimethylmethoxysilan und/oder 4-Aminobutyltriethoxysilan als Aminosilan-Kupplungsmittel.

2. Verfahren nach Anspruch 1, dadurch gekennzeichnet, daß ein Schamottestein oder ein Schamottesteinmaterial als Träger verwendet werden, die durch Hochtemperaturbehandlung eines Diatomeenerde enthaltenden Materials erhalten sind.

3. Verfahren nach Anspruch 1 oder 2, dadurch gekennzeichnet, daß bifunktionelle Aldehyde als multifunktionelle Aldehyde eingesetzt werden.

4. Verfahren nach einem der Ansprüche 1 bis 3, dadurch gekennzeichnet, daß Glyoxal und/oder Glutaraldehyd als multifunktionelle Aldehyde eingesetzt werden.

5. Immobilisierte Alkoholoxidasen, erhältlich nach dem Verfahren nach einem der Ansprüche 1 bis 4, vorzugsweise in Form einer an der Oberfläche des Trägers gebundenen Enzym-Monoschicht.

6. Verwendung der immobilisierten Alkoholoxidase nach Anspruch 5 zur Erzeugung von Aldehyden, zur Entfernung von Alkohol und/oder zur Messung von Alkoholkonzentrationen.

7. Vorrichtung vom Strömungstyp zur Messung von Alkohol, die eine mit immobilisierter Alkoholoxidase gefüllte Säule (4) und eine Wasserstoffperoxidelektrode (6), die stromab von der Säule (4) vorgesehen ist, aufweist, gekennzeichnet durch eine immobilisierte Alkoholoxidase nach Anspruch 5.

8. Vorrichtung vom Strömungstyp zur Messung von Alkohol nach Anspruch 7, dadurch gekennzeichnet, daß sie zusätzlich eine stromauf von der Säule (4) mit der immobilisierten Alkoholoxidase oder stromab von der Wasserstoffperoxidelektrode (6) vorgesehene Enzymelektrode (14) mit immobilisiertem Enzym aufweist.

## Revendications

1. Procédé de préparation d'une alcool-oxydase immobilisée, comprenant au moins les étapes de :
(A) traitement de la surface d'un support, de préférence d'un support poreux, avec un agent de couplage aminosilane, pour former des groupes amino sur la surface du support ;
(B) liaison d'un aldéhyde multifonctionnel à des groupes amino formés dans l'étape (A) sur la surface du support,
(C) lavage du support pour retirer l'aldéhyde multifonctionnel qui n'a pas réagi, et
(D) liaison d'une alcool-oxydase à des groupes aldéhyde de l'aldéhyde multifonctionnel lié aux groupes amino,
caractérisé en ce que
- une terre de diatomées ou un brique réfractaire ou un matériau de brique réfractaire est utilisé comme support,
et
- du γ-aminopropyltriéthoxysilane, du 4-aminobutyldiméthylméthoxysilane et/ou du 4-aminobutyltriéthoxysilane sont utilisés comme agents de couplage aminosilanes.

2. Procédé selon la revendication 1, caractérisé en ce qu'une brique réfractaire ou un matériau de brique réfractaire obtenu par traitement à haute température d'un matériau contenant une terre de diatomées est utilisé comme support.

3. Procédé selon la revendication 1 ou 2, caractérisé en ce que des aldéhydes bifonctionnels sont utilisés comme aldéhydes multifonctionnels.

4. Procédé selon l'une des revendications 1 à 3, caractérisé en ce que du glyoxal et/ou du glutaraldéhyde sont utilisés comme aldéhydes multifonctionnels.

5. Alcool-oxydases immobilisées pouvant être obtenues par le procédé selon l'une des revendications 1 à 4, de préférence sous forme d'une monocouche d'enzyme liée à la surface du support.

6. Utilisation de l'alcool-oxydase immobilisée selon la revendication 5 pour la production d'aldéhydes, pour le retrait d'alcools et/ou pour la mesure de concentrations d'alcools.

7. Appareil de mesure d'alcools du type à écoulement, comprenant une colonne (4) remplie d'alcool-oxydase immobilisée, et une électrode à peroxyde d'hydrogène (6) prévue en aval de la colonne (4), caractérisé par une alcool-oxydase immobilisée selon la revendication 5.

8. Appareil de mesure d'alcools du type à écoulement selon la revendication 7, caractérisé en ce qu'il comprend en outre une électrode à enzyme immobilisée (14) pour détecter une concentration de glucose, prévue en amont de la colonne (4) contenant une alcool-oxydase immobilisée ou en aval de l'électrode à peroxyde d'hydrogène (6).
